# FASCICULE DE BREVET EUROPEEN

(11) **EP 0 640 360 B1**
(45) Date de publication et mention de la délivrance du brevet: **08.03.2000**
(21) Numéro de dépôt: 94401760.7
(22) Date de dépôt: 01.08.1994
(51) Int. Cl.: A61N 1/37

(54) **Circuit de détection pour détecteur de tachyarythmie**
Schaltung zur Feststellung von Tachyarrhythmien
Detection circuit for tachyarrhythmia detector

(30) Priorité: 13.08.1993 FR 9309950
(43) Date de publication de la demande: 01.03.1995
(73) Titulaire: ELA MEDICAL (Société anonyme), F-92541 Montrouge Cédex (FR)
(72) Inventeur: Jacobson, Peter, F-67500 Haguenau (FR); Kroiss, Daniel, F-67590 Schweighouse-Moder (FR)
(74) Mandataire: Laget, Jean-Loup

(56) Documents cités:
- EP-A- 0 549 438
- WO-A-89/03705
- US-A- 4 708 144
- US-A- 5 117 824
- US-A- 5 188 117

## Description

L'invention concerne des circuits de détection pour détecteurs de tachyarythmie et plus particulièrement des circuits de détection pour des détecteurs de tachyarythmie implantables utilisant des électrodes appliquées sur le myocarde ou à proximité de celui-ci, appelées électrodes cardiaques directes, par opposition aux électrodes externes appliquées sur la peau pour la détection de l'activité cardiaque électrique. Les défibrillateurs, les appareils de cardioversion, les stimulateurs cardiaques anti-tachycardiques, automatiques et implantables, ou les dispositifs qui combinent deux ou plusieurs de ces fonctions, ont besoin de détecteurs de tachyarythmie. L'objectif du circuit de détection est de détecter et signaler l'occurrence d'une dépolarisation cardiaque dans le tissu adjacent à une ou plusieurs électrodes en contact avec le coeur ou à proximité du coeur.

Il est essentiel d'isoler l'amplificateur de détection pour le protéger vis-à-vis d'une saturation due aux impulsions de stimulation, et de prévoir d'autres types de protection contre les dommages ou les dysfonctionnements causés par des sources d'interférence externes. Ces techniques sont familières à l'homme de l'art et hors du domaine de l'invention. De même il est important de prévoir un comparateur avec des seuils de détection symétriques positif et négatif, ou un redressement à double alternance et un comparateur unique , pour détecter les deux polarités des signaux. Ces techniques sont également familières pour l'homme de l'art et également hors du domaine de l'invention. Le domaine de l'invention est la sélection des caractéristiques du filtre et du seuil du comparateur. Elle peut être également appliquée à des systèmes comprenant une période réfractaire absolue additionnelle (blanking), une protection vis-à-vis d'interférences, un redressement et/ou des seuils symétriques du comparateur.

Le champ d'application de l'invention comprend les circuits de détection de l'arythmie auriculaire ou ventriculaire.

Le brevet US No 4 184 493 au nom de Langer (déposé en fév. 1978) décrit un circuit de détection pour un défibrillateur implantable, comprenant un amplificateur, un filtre passe-haut du premier ordre (à fréquence de coupure non décrite), un comparateur bidirectionnel et un circuit qui mesure la fraction de temps durant lequel le signal est validé par le comparateur. Le détecteur mesure finalement le rapport moyen entre pente forte et pente faible de l'ECG. Le circuit comprend une contre-réaction pour ajuster automatiquement le gain de l'amplificateur en vue de maintenir constante l'amplitude à la sortie du filtre.

Le brevet US No 4 393 877 au nom de Imran (déposé en mai 1981) décrit un circuit de détection pour un défibrillateur implantable, qui comprend une détection de pente et une détection de passage par zéro. Le détecteur de pente comprend un amplificateur, un filtre passe-haut du premier ordre (à fréquence de coupure non décrite), un comparateur unidirectionnel et un multivibrateur monostable pour le minutage d'une période réfractaire. Le circuit de Imran comprend une contre-réaction pour ajuster automatiquement le gain de l'amplificateur en vue de maintenir constante l'amplitude à l'entrée du filtre.

Le brevet US No 4 559 946 au nom de Mower (déposé en juin 1982) décrit également un circuit de détection synchronisant les chocs à l'activité cardiaque dans un défibrillateur implantable, qui comprend un circuit de détection de pente, suivi par un comparateur. Le circuit de Mower comprend une contre-réaction pour ajuster automatiquement le seuil du comparateur à une fraction de l'amplitude crête récente à la sortie du filtre.

Le brevet US No 4 819 643 an nom de Menken (déposé en nov. 1986) décrit un défibrillateur avec fonctions de stimulation et de détection, et un canal séparé de détection de fibrillation à contrôle automatique de gain, qui retarde la stimulation jusqu'à ce que le gain du canal de détection de fibrillation atteigne sa sensibilité maximale. Menken ne donne pas de détails sur la bande passante de l'amplificateur.

Le brevet US No 4 940 054 au nom de Grevis (déposé en avril 1988) décrit un défibrillateur avec au moins deux valeurs de sensibilité, commutées en fonction du type de rythme détecté. Grevis ne donne pas non plus de détails sur la bande passante de l'amplificateur.

Le brevet US No 4 880 004 au nom de Baker (déposé en juin 1988) décrit un circuit de détection de l'arythmie, qui comprend un amplificateur d'entrée à bande passante non spécifiée, suivi par un canal de détection comprenant un filtre passe-haut à 25 Hz, et un canal de mesure à filtrage passe-haut additionnel à 12 Hz, chaque canal étant suivi par des comparateurs. Le circuit de Baker comprend une contre-réaction pour ajuster automatiquement le gain de l'amplificateur en vue de maintenir constante la marge de détection (c'est-à-dire la différence entre le signal dans le canal de détection et son seuil). En raison du filtrage additionnel dans le canal de mesure, cette marge améliore la distinction aux basses fréquences.

Le brevet US No 4 967 747 au nom de Carroll (déposé en mai 1989) décrit un défibrillateur implantable avec un circuit de détection, consistant en un bloc gain/filtre à capacité commutée, à bande passante non spécifiée, prévu pour fournir des modifications de gain sans transitoires pour le contrôle automatique de gain.

Le brevet US No 5 117 824 au nom de Keimel (déposé en nov. 1990) décrit un circuit implantable de stimulation et de détection à contrôle automatique de seuil, qui n'ajuste pas le seuil après la stimulation. Keimel ne donne pas de détails sur la bande passante de l'amplificateur.

Il y a lieu d'améliorer les circuits de détection des défibrillateurs implantables. Dans "State-of-the-Art of the AICD", Pace, Mai 1991, Winkle décrit un défibrillateur sans contrôle automatique de gain, pour lequel la programmation de la sensibilité demande beaucoup de soins au moment de l'implantation et pendant le suivi pour être certain que les ondes T ne soient pas sur-détectées, et ce qui est plus important encore, pour que la fibrillation/tachycardie ventriculaire ne soit pas sous-détectée".

Dans "Failure of a Second and Third Generation Implantable Cardioverter to Sense Ventricular Tachycardia: Implications for Fixed-Gain Sensing Devices", Pace, mai 1992, Sperry et al. ont écrit que "l'on a besoin de circuits à gain fixe plus sensibles ou d'une détection à contrôle automatique de gain pour détecter les signaux de faible amplitude sur une base stable. L'aspect indésirable d'une telle sensibilité élevée est celui de la sur-détection (par exemple des ondes T) qui se traduit par une augmentation du risque de décharges inappropriées. Le défaut de détection de la fibrillation ventriculaire a cependant été également signalé avec des dispositifs à gain automatique".

Il est clair qu'un circuit de détection de tachyarythmie doit pouvoir détecter des dépolarisations cardiaques mais rejeter les signaux indésirables, comprenant la repolarisation cardiaque (onde T), et comprenant des signaux émanant du retour à l'équilibre du système d'électrodes et du circuit de sortie du stimulateur cardiaque après la stimulation, appelés artefacts de stimulation. Les techniques de l'art antérieur consistent en des filtres passe-haut, des périodes réfractaires et un contrôle automatique de gain ou de seuil ; mais des insuffisances existent dans chacun de ces cas.

Insuffisances des filtres passe-haut de l'art antérieur: un filtre pour un dispositif implantable d'assistance à la vie doit être d'une complexité minimale nécessaire pour satisfaire aux deux critères de performance: une faible probabilité de rejet des signaux désirés et de détection de signaux non désirés. Aucun des brevets de l'art antérieur ne décrit un procédé systématique pour la sélection des caractéristiques du filtre telles que l'ordre, le facteur de qualité Q, et les fréquences de coupure, pour satisfaire aux critères de performance avec un matériel de complexité minimale.

Insuffisances des périodes réfractaires: si les concepteurs sélectionnent une longueur de période réfractaire suffisamment courte pour permettre la détection de tachyarythmies, il apparaît alors que la période réfractaire ne couvre pas les ondes T quand la fréquence cardiaque est faible (la durée entre dépolarisation et repolarisation augmente à mesure que diminue la fréquence cardiaque). Si les périodes réfractaires étaient suffisantes à elles seules, tous les dispositifs de l'art antérieur ne prévoiraient pas un contrôle automatique additionnel complexe de gain ou de seuil.

Insuffisances du contrôle automatique de gain ou de seuil: les détecteurs peuvent améliorer la sélectivité (probabilité de rejet des signaux indésirables) et la sensibilité (probabilité de détection des signaux désirés) avec cette technique, seulement quand les caractéristiques d'événements récemment détectés prédisent correctement les mêmes caractéristiques d'événements futurs. Bien que les concepteurs de stimulateurs cardiaques de bradycardie savent que l'amplitude d'un battement cardiaque normal (onde R) prédit l'amplitude de l'onde R suivante avec une bonne corrélation, les concepteurs de détecteurs de tachycardie ont besoin de poser deux questions additionnelles qui ne l'avaient pas été dans l'art antérieur:
- dans quelle mesure l'amplitude du signal désiré (dépolarisation en tachyarythmie) prédit l'amplitude du signal désiré suivant ?
- dans quelle mesure l'amplitude du signal désiré (dans ce cas également la dépolarisation en tachyarythmie) prédit l'amplitude du signal non désiré suivant (repolarisation) ?

Pour répondre à la première question, on considère la nature de la fibrillation ventriculaire, détectée par des électrodes bipolaires placées directement sur la surface du coeur. Le détecteur ne peut risquer de manquer la détection d'une fibrillation ventriculaire, qui est la plus mortelle des tachyarythmies. Les détecteurs implantables de tachyarythmie utilisent normalement des électrodes bipolaires endocavitaires ou épicardiques du fait de leur bonne réjection des champs lointains par comparaison avec des électrodes unipolaires.

La fibrillation ventriculaire consiste en des "ondelettes vagabondes", multiples fronts de dépolarisation de faible amplitude se déplaçant de façon aléatoire sur de la surface du coeur dans toutes les directions. L'amplitude détectée quand une ondelette passe devant des électrodes bipolaires dépend de la composante du vecteur de l'ondelette qui se situe dans l'axe des électrodes bipolaires (une ondelette circulant perpendiculairement à l'axe des électrodes produit une amplitude nulle du fait qu'elle atteint simultanément les deux électrodes; inversement, une ondelette circulant dans l'axe produit une amplitude maximale du fait qu'elle atteint d'abord une électrode et ensuite l'autre). Des mesures concernant la fibrillation ventriculaire ou la tachycardie ventriculaire multifocale humaines détectées par des électrodes bipolaires montrent une corrélation presque nulle entre l'amplitude d'un complexe détecté et le suivant.

Pour répondre à la seconde question, les inventeurs ont mesuré l'amplitude de l'onde R et de l'onde T chez vingt patients avec des électrodes bipolaires endocavitaires. La mesure de la corrélation entre l'amplitude de l'onde T et l'amplitude de l'onde R n'a été que de 50%.

Pour quelle raison, face à ces faits, de nombreuses conceptions de l'art antérieur font-elles appel à un contrôle automatique de gain ? Tout d'abord parce qu'il existe néanmoins quelques petites corrélations, de sorte que cette technique peut statistiquement améliorer un peu les performances. Peut-être également parce que de nombreuses publications ont observé que la fibrillation ventriculaire détectée sur la surface du corps (et non sur la surface du coeur) diminue en amplitude à mesure que se poursuit la fibrillation. Ceci est dû au fait que les "ondelettes vagabondes" deviennent plus petites et plus désorganisées, ce qui fait que leur somme électrique, vue à la surface, diminue avec le temps. Cependant, un concepteur ne doit pas s'attendre à ce qu'il en aille de même avec des électrodes cardiaques directes.

Il est donc clair qu'un contrôle automatique de gain ou de seuil, et des périodes réfractaires, ne peuvent pas fournir une solution complète au problème de la détection de la tachyarythmie avec des électrodes cardiaques directes. Le concepteur doit également optimiser systématiquement le filtrage pour améliorer la sensibilité et la sélectivité; mais aucun art antérieur n'enseigne la manière de le faire.

Comme le circuit de détection forme une partie d'un dispositif implantable, le concepteur doit minimiser la consommation électrique pour prolonger la durée de vie de la pile, et minimiser la complexité pour réduire les dimensions de l'implant. En général, dans le domaine de la conception de micro-circuits, l'augmentation du gain ou de la bande passante d'un amplificateur et la combinaison avec un filtre entraînent une augmentation corrélative de la consommation électrique. Ainsi, le circuit de détection doit fonctionner avec un produit gain * bande passante minimal, et un nombre de composants minimal.

Le but de l'invention est donc de proposer d'optimiser la conception d'un circuit de détection de tachyarythmie prévu pour être utilisé avec des électrodes cardiaques directes, en particulier dans des dispositifs implantables. Avec le circuit de l'invention une méthode quantitative peut être mise en oeuvre pour examiner les effets des paramètres de conception sur l'un ou plusieurs des critères suivants: sensibilité, sélectivité, produit gain - bande passante, et nombre de composants.

Un autre but encore de l'invention est d'appliquer l'optimisation à la conception d'un circuit de détection pour détecter les tachyarythmies ventriculaires et rejeter les ondes T et les artefacts de stimulation. Dans son application à ce problème, l'invention prévoit :
- un filtre passe-haut du troisième ordre, avec une fréquence de coupure d'approximativement 16 Hz;
- un comparateur relié à la sortie du filtre, avec un seuil d'approximativement 0,40 mV, dans la bande passante, rapporté à l'entrée du filtre.

L'invention a pour objet un circuit de détection pour détecteur de tachyarythmie comprenant un filtre passe-haut, et un comparateur à seuil relié à la sortie du filtre, caractérisé en ce que le filtre est du troisième ordre et présente une fréquence de coupure d'approximativement 16 Hz.

Selon l'invention :
- ledit comparateur a un seuil d'approximativement 0,40 mV rapporté à l'entrée du filtre, dans la bande passante;
- ledit seuil est programmable ou est ajusté automatiquement, dans une plage comprenant 0,40 mV.

La figure 1 est une vue d'ensemble du circuit de détection constitué selon l'invention.

La figure 2 est une vue d'ensemble du procédé qui peut être effectué avec la circuit de l'invention.

La figure 3 est une courbe d'optimisation de la fréquence de coupure du filtre et du seuil du comparateur, avec un filtre du troisième ordre, selon le procédé de l'invention.

La figure 4 est un diagramme schématique du filtre.

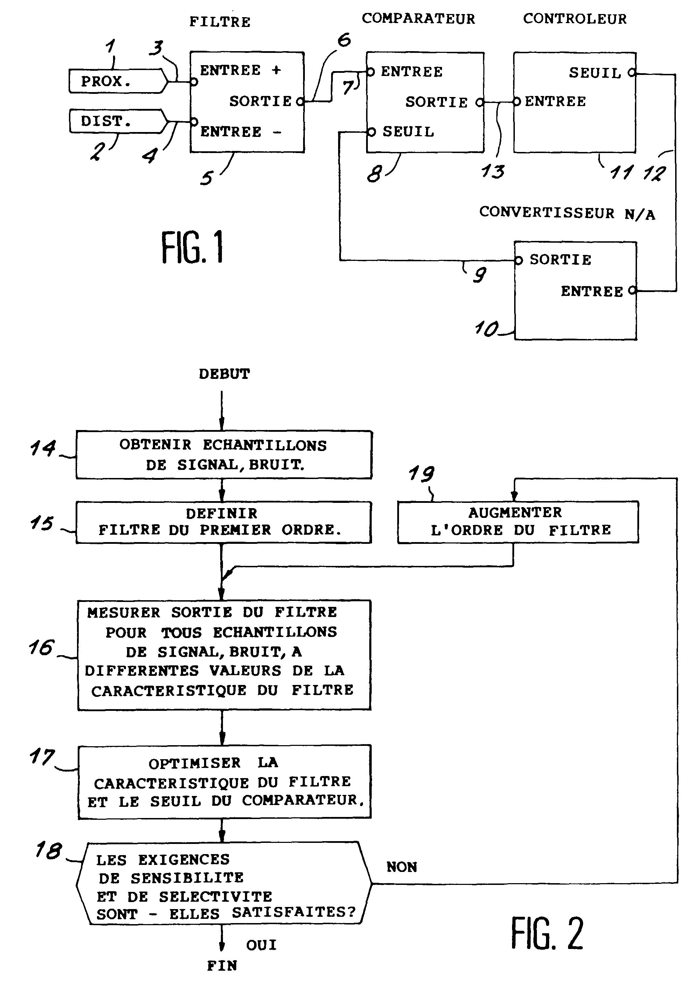

La figure 1 montre un circuit de détection destiné à un détecteur de tachyarythmie selon l'invention. Les bornes 1 et 2 des électrodes cardiaques directes bipolaires appliquent le signal provenant du coeur aux entrées différentielles 3, 4 du filtre 5. Le filtre 5 est optimisé sur le plan de sa sensibilité et de sa sélectivité par le procédé de l'invention comme décrit ci-dessous. Sa sortie 6 est reliée à une entrée 7 d'un comparateur 8. L'autre entrée 9 du comparateur 8 est reliée à une tension de référence fournie, dans le mode de réalisation préféré, par un convertisseur numérique-analogique 10.

Toujours en se référant à la figure 1, un contrôleur 11 présente une sortie numérique 12 pour définir le seuil de détection 9 du comparateur par l'intermédiaire du convertisseur numérique-analogique 10. Le contrôleur 11 reçoit également la sortie 13 du comparateur 8, indiquant, lorsqu'elle est activée, que le circuit de détection détecte une dépolarisation cardiaque. Le contrôleur 11 peut fournir des périodes réfractaires, et par l'intermédiaire du convertisseur numérique-analogique 10, il peut également fournir un contrôle automatique de seuil et/ou un contrôle programmable de seuil , ainsi que cela est connu dans l'art antérieur.

La figure 2 montre le procédé pour optimiser les paramètres du circuit de détection montré à la figure 1. Le concepteur obtient d'abord des échantillons des signaux désirés à détecter, et des signaux non désirés, à rejeter, en 14. Les signaux désirés comprennent les dépolarisations cardiaques provenant de la fibrillation ventriculaire et de la tachycardie ventriculaire, alors que les signaux non désirés comprennent les repolarisations cardiaques (ondes T) et les artefacts de stimulation.

En se référant toujours à la figure 2, le concepteur cherche ensuite à satisfaire aux besoins avec un simple filtre du premier ordre en 15. En 16, le concepteur mesure la sortie du filtre pour chacun des échantillons désirés et non désirés, puis répète ces mesures pour des valeurs différentes d'un paramètre du filtre tel que la fréquence de coupure du filtre. En 17, le concepteur prend ces données et les utilise pour optimiser la caractéristique du filtre et le seuil du comparateur 8.

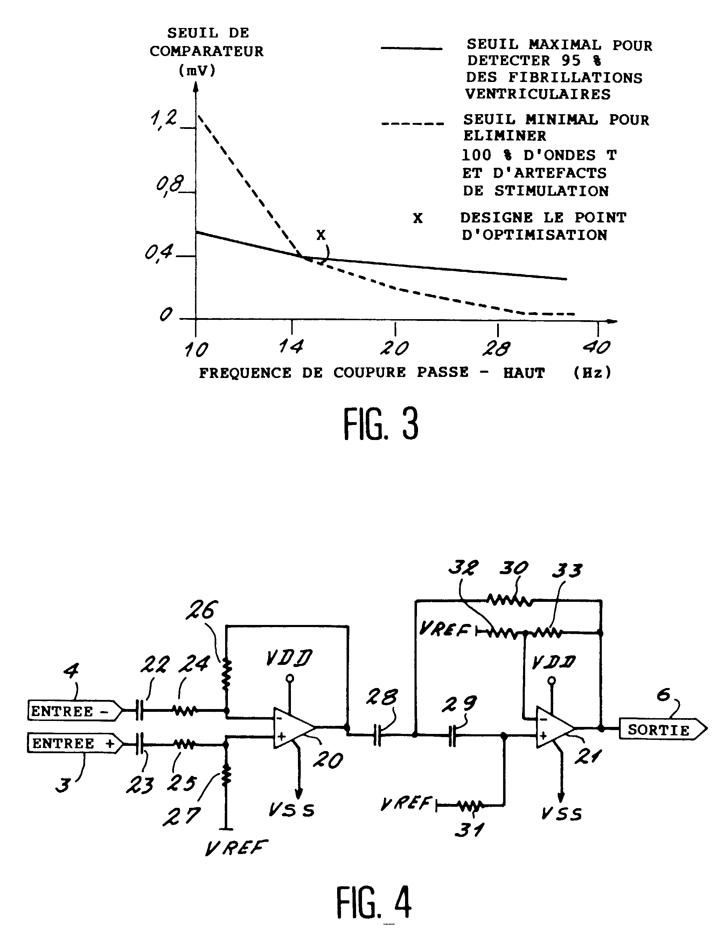

Une technique d'optimisation montrée à la figure 3 consiste à établir la courbe du seuil le plus bas du comparateur qui rejette avec succès le pourcentage requis de signaux non désirés, et le seuil le plus élevé du comparateur qui détecte avec succès le pourcentage requis de signaux désirés, les deux en fonction de la caractéristique du filtre qui a été modifiée à l'étape 16 de la figure 2. L'exemple de la figure 3 exige le rejet de 100% des signaux non désirés et la détection de 95% des signaux désirés. Tous les points du graphique qui sont au-dessus du seuil de rejet le plus bas, et également au-dessous du seuil de détection le plus élevé, représentent des combinaisons de seuil du comparateur et de caractéristique du filtre qui satisfont aux exigences de sensibilité et de sélectivité.

Après avoir généré la courbe de la figure 3, le concepteur doit sélectionner une valeur optimale de la caractéristique du filtre et du seuil correspondant du comparateur, dans l'ensemble des points acceptables. Selon une variante préférée du procédé, le concepteur sélectionne le point ayant la valeur la plus basse du produit gain ∗ bande passante. Dans l'exemple de la figure 3, il s'agit du point avec le seuil du comparateur le plus élevé qui satisfait aux besoins de sensibilité et de sélectivité (c'est-à-dire au-dessous de la ligne continue et au-dessus de la ligne discontinue).

En revenant à la figure 2, il peut arriver en 17 que le concepteur ne puisse pas trouver de combinaison de la caractéristique du filtre et du seuil du comparateur qui satisfasse aux exigences de sensibilité et de sélectivité (la ligne discontinue se trouvant toujours au-dessus de la ligne continue) et en 18 le concepteur décide d'augmenter l'ordre du filtre 19 puis revient à l'étape 16 pour essayer d'obtenir l'optimisation avec ce filtre plus compliqué. L'augmentation de l'ordre du filtre augmente le nombre de composants.

Ainsi, en suivant le procédé de l'invention, le concepteur obtient un circuit de détection optimisé de complexité minimale avec un produit gain ∗ bande passante minimal. Les inventeurs ont appliqué le procédé de l'invention au problème de la détection de la tachycardie et de la fibrillation ventriculaires tout en rejetant l'onde T, avec l'exigence du rejet de 100% des ondes T et de la détection de 95% de toutes les dépolarisations vraies. Les inventeurs ont utilisé la fréquence de coupure passe-haut du filtre en tant que caractéristique variable du filtre à l'étape 17

La figure 3 montre qu'un filtre du troisième ordre convient pour la sensibilité et la sélectivité, et est optimisé pour le produit gain ∗ bande passante avec une fréquence de coupure passe-haut d'approximativement 16 Hz et un seuil de comparateur dans la bande passante, rapporté à l'entrée, d'approximativement 0,40 mV.

La figure 4 montre la constitution du filtre dérivé de l'utilisation du procédé de l'invention. Les circuits d'alimentation de puissance non montrés donnent VDD = 0,0V, VSS = -3,0 V, et VREF = - 0,80 V. Le filtre consiste en un étage du premier ordre utilisant un amplificateur 20, et un étage du second ordre utilisant un amplificateur 21. Le gain du premier étage est d'approximativement 160, avec une unique coupure passe-haut à 16 Hz. Le gain du second étage est d'approximativement 1,6, avec une réponse de Butterworth passe-haut du second ordre, également avec une coupure à 16 Hz. Le tableau ci-dessous donne les valeurs typiques des résistances et des condensateurs du filtre.

| **Tableau montrant les valeurs typiques des composants** | | | |
|---|---|---|---|
| Composant | type | valeur | notes |
| 22, 23 | condensateur | 0,10 µF | non polarisé |
| 24, 25 | résistance | 0,10 MOhm | |
| 26, 27 | résistance | 16 MOhm | |
| 28, 29 | condensateur | 1,0 nF | non polarisé |
| 30, 31 | résistance | 10 MOhm | |
| 32 | résistance | 15 MOhm | |
| 33 | résistance | 8,2 MOhm | |

La figure 4 montre un filtre classique à résistances et condensateurs discrets, bien qu'un filtre présentant les mêmes caractéristiques puisse être intégré en utilisant les techniques de capacités commutées ou des techniques de filtres numériques, connues habituellement des concepteurs qualifiés.

Des caractéristiques telles qu'une protection vis-à-vis de sources de haute énergie telles que des défibrillateurs, ou vis-à-vis de sources haute fréquence telles qu'utilisées en électrochirurgie, ne sont pas montrées car elles ne concernent pas l'invention et sont également connues.

La paire de condensateurs 22, 23 et les paires de résistances 24, 25 et 26, 27 doivent être étroitement adaptées les unes aux autres pour un bon rejet du mode commun.

Bien que l'invention ait été décrite en référence à un mode de réalisation particulier, il doit être compris que ce mode de réalisation est simplement illustratif de l'application des principes de l'invention. De nombreuses autres modifications peuvent être apportées et d'autres agencements peuvent être conçus sans s'écarter du champ d'application de la présente invention.

## Revendications

1. Circuit de détection pour détecteur de tachyarythmie, comprenant un filtre passe-haut (5) et un comparateur à seuil (8) relié à la sortie du filtre (5), caractérisé en ce que le filtre (5) est du troisième ordre, et présente une fréquence de coupure d'approximativement 16 Hz.

2. Circuit de détection selon la revendication 1, caractérisé en ce que ledit comparateur (8) a un seuil d'approximativement 0,40 mV rapporté à l'entrée du filtre (5), dans la bande passante.

3. Circuit de détection selon la revendication 2, caractérisé en ce que ledit seuil est programmable ou est ajusté automatiquement, dans une plage comprenant 0,40 mV.

## Claims

1. A detection circuit for a tachyarhythmia detector, comprising a high-pass filter (5) and a threshold comparator (8) connected to the output of the filter (5), characterised in that the filter (5) is of the third order and presents a cut-off frequency of approximately 16 Hz.

2. The detection circuit of claim 1, characterised in that said comparator (8) has a threshold value of approximately 0.40 mV referred to the input of the filter (5), in the pass-band.

3. The detection circuit of claim 2, characterised in that said threshold value is programmable or automatically adjusted, within a range comprising 0.40 mV.

## Patentansprüche

1. Erfassungsschaltung für einen Tachyarrythmie-Erfasser, mit einem Hochpass-Filter (5) und einem mit dem Ausgang des Filters (5) verbundenen Schwellenkomparator (8), dadurch gekennzeichnet, dass das Filter (5) ein Filter dritter Ordnung ist und eine Grenzfrequenz von ungefähr 16 Hz aufweist.

2. Erfassungsschaltung nach Anspruch 1, dadurch gekennzeichnet, dass der Komparator (8) eine am Eingang des Filters (5) im Durchlassbereich vorgesehene Schwelle von ungefähr 0,40 mV hat.

3. Erfassungsschaltung nach Anspruch 2, dadurch gekennzeichnet, dass die Schwelle in einem Bereich, der 0,40 mV umfasst, programmierbar oder automatisch angepasst ist.
